# EUROPEAN PATENT APPLICATION

(11) **EP 2 444 398 A2**
(43) Date of publication of application: **25.04.2012**
(21) Application number: 10789629.2
(22) Date of filing: 29.04.2010
(51) Int. Cl.: C07D 401/06, A61K 31/4196, A61K 31/506, A61K 47/40

(54) **NOVEL INTERMEDIATES OF VORICONAZOLE AND PREPARATION METHOD OF VORICONAZOLE USING THE SAME**

(30) Priority: 17.06.2009 KR 20090053670
(71) Applicant: Boryung Pharmaceutical Co., Ltd, Jongno-gu Seoul 110-750 (KR)
(72) Inventor: CHOI, Ok Kyoung, Suwon-si Gyeonggi-do 440-722 (KR); PARK, Yong, Gwacheon-si Gyeonggi-do 427-060 (KR); LEE, Joon Kwang, Gwangmyeong-si Gyeonggi-do 423-774 (KR); KIM, Ji Han, Seoul 150-823 (KR); TAN, Hyun Kwang, Seoul 137-952 (KR)
(74) Representative: von Kreisler Selting Werner
(86) International application number: PCT/KR2010/002715
(87) International publication number: WO 2010/147302

(57) **Abstract**

The present invention provides a compound of formula 2 and a compound of formula 4 which are a novel intermediate of voriconazole and a process for the preparation of voriconazole using the same. The novel intermediates of the present invention are environmentally-friendly due to no emission of irritative and offensive odor unique to thiols and enable the production of voriconazole with high purity and high yield.

## Description

### TECHNICAL FIELD

The present invention relates to a novel intermediate of voriconazole and a process for the preparation of voriconazole using the same.

### BACKGROUND ART

Voriconazole is a triazole-based antifungal agent disclosed in Korean Patent Application Publication No. 1993-0011039 B1 and is a broad-spectrum antifungal agent which exhibits antifungal activity against various *Candida* spp. including fluconazole-resistant *Candida krusei, Candida glabrata* and *Candida albicans* and fungicidal activity against all of *Aspergillus* spp. Voriconazole has the chemical name of (2R,3S)-2-(2,4-difluorophenyl)-3-(5-fluoropyrimidin-4-yl)-1-(1H-1,2,4-triazol-1-yl)butan-2-ol and is represented by the structural formula 1 below. Voriconazole is currently marketed under the trade name of VFEND^{™} by Pfizer Inc.

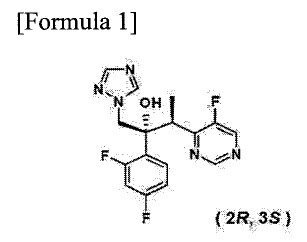

As shown in the above structural formula, voriconazole has two asymmetric carbon atoms. Therefore, two diastereoisomeric pairs are involved in the synthesis of voriconazole, and the presence of two optical stereoisomers in each diastereoisomer finally gives rise to four stereoisomers.

For this reason, voriconazole is prepared by separating an enantiomeric pair having a preferred configuration (2R,3S/2S,3R) from among four stereoisomers, and then optically resolving only the pure (2R,3S)-stereoisomer using an optically active acid, R-(-)-10-camphorsulfonic acid. Such a known process for the preparation of voriconazole is as follows.

Korean Patent Application Publication No. 1993-0011039 B1 (hereinafter, referred to as Patent No. 0011039), as shown in Reaction Scheme 1 below, discloses a process for the preparation of voriconazole in which four isomers are synthesized by reacting 4-chloro-6-ethyl-5-fluoropyrimidine and 1-(2,4-difluorophenyl)-2-(1H-1,2,4-triazol-1-yl)ethanone using lithium diisopropylamide (LDA) which is a strong base, column chromatographic separation is carried out to yield a desired enantiomeric pair (2R,3S/2S,3R), a reaction to remove the chlorine substituent is carried out in the presence of hydrogen gas and palladium, and then the resulting product is subjected to optical resolution using camphorsulfonic acid. However, this production process is not feasible for industrial production applications because the process is complicated due to a need for column chromatography to obtain an enantiomeric pair after the reaction and it is positively necessary to maintain an ultralow temperature of below -50□ in order to maintain stability of an organic lithium salt formed during the reaction. Further, since the reaction using such organic lithium is low in a total yield of less than 50% and exhibits no selectivity of a diastereoisomeric pair, a theoretical yield of a preferred enantiomeric pair (2R,3S/2S,3R) is not greater than 25%. In fact, the yield of an enantiomeric pair (2R,3S/2S,3R) by way of this process ranges from 12 to 25%. Further, a process of removing the chlorine substituent using expensive palladium and a hydrogen reaction with high inflammability after the separation of enantiomers has a limitation in terms of industrial application.

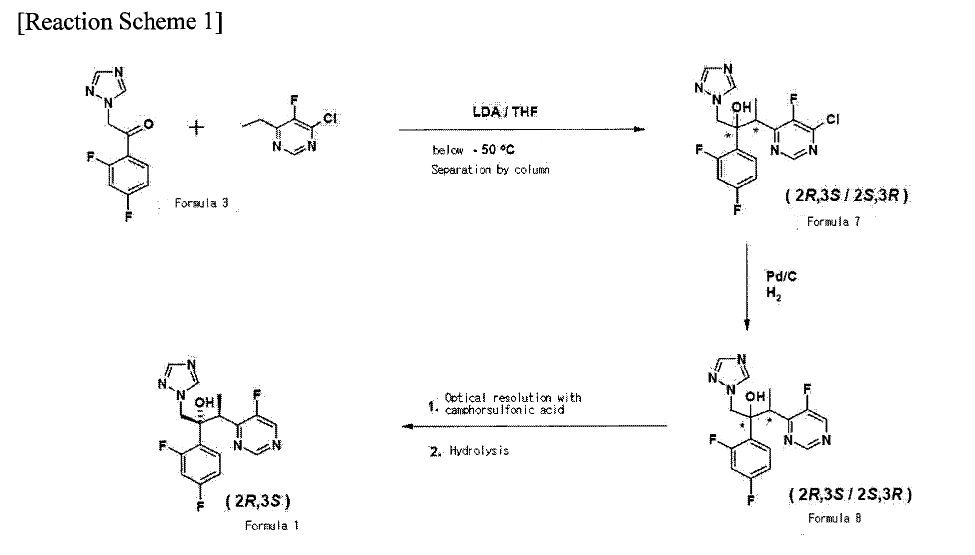

International Publication No. WO 2006/065726 discloses a process for preparing a desired enantiomeric pair (2R,3S/2S,3R) of voriconazole in which only the conditions of a reaction solvent are different from those described in Korean Patent Application Publication No. 1993-0011039 B1 and which does not involve column chromatographic purification. However, the process of WO 2006/065726 still has a low yield of 26% and has disadvantages of conventional production processes, such as subsequent reaction to remove the chlorine substituent in the presence of palladium and high-pressure hydrogen.

Further, International Publication No. WO 2007/013096 also discloses a process for preparing a desired enantiomeric pair of voriconazole in which an ultralow temperature of below -50□ is maintained as shown in Reaction Scheme 1 above, an organic lithium salt is used and a solvent composition is changed to a mixed solvent of tetrahydrofuran and n-hexane. However, a yield of the product through the crystallization method of this reaction is not more than 30%, and the ratio of an isomeric pair is also merely (2R,3S/2S,3R):(2R,3R/2S,3S)= 75:25. Further, since the removal of the chlorine substituent in the presence of Raney-nickel and hydrogen gas subsequent to this reaction is also a conventional method using an expensive metal catalyst such as palladium and a high-pressure hydrogen reaction is necessary as in the conventional method, there is a limitation in terms of industrial application.

Korean Patent No. 10-0269048 addresses an improvement of conventional problems encountered in the reaction using an organic lithium salt and discloses a process for the preparation of voriconazole, which enhances stereoselectivity of an isomeric pair and improves a reaction yield through the Reformatsky reaction using an activated zinc metal, as shown in Reaction Scheme 2 below. Specifically, a (2R,3S/2S,3R) enantiomeric pair and a (2R,3R/2S,3S) enantiomeric pair of the product are formed in a molar ratio of 9:1 in terms of selectivity after the Reformatsky reaction, total 4 unseparated isomers in the form of a hydrochloride are separated in a 65% yield by way of crystallization separation, the chlorine substituent is removed using a palladium metal and hydrogen gas, and then a desired isomeric pair (2R,3S/2S,3R) is separated. Thereafter, optical resolution is carried out by separating only the pure (2R,3S) stereoisomer using R-(-)-10-camphorsulfonic acid as in a conventional method.

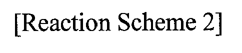

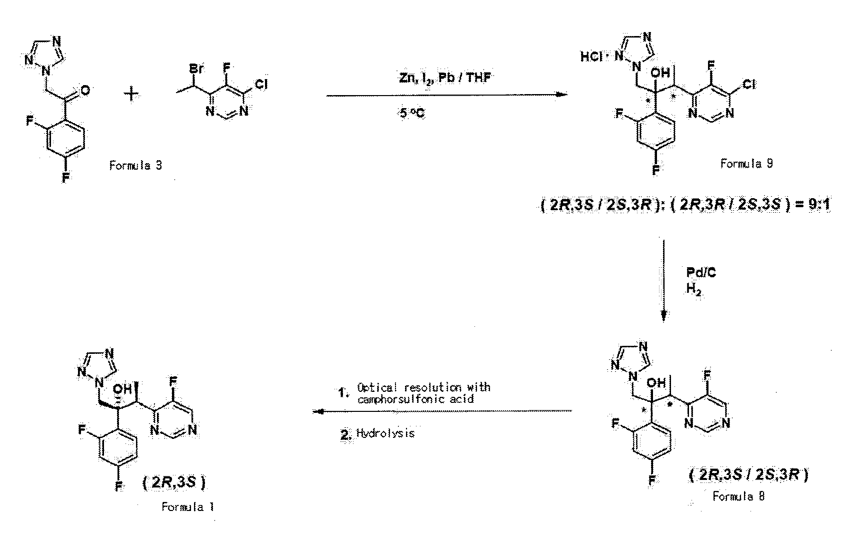

This reaction does not need maintenance of an ultralow temperature and exhibits a significantly improved stereoselectivity of 9:1 of a (2R,3S/2S,3R) enantiomeric pair and a (2R,3R/2S,3S) enantiomeric pair as compared to 1:1 of Korean Patent Application Publication No. 1993-0011039 B1, in conjunction with an improved yield of 65%, thus being industrially applicable. However, as disclosed in the literature (Organic Process Research & Development 2001, 5, 28-36, by the former developer Pfizer Inc.), the compound of formula 10 (approximately 10%) produced during the reaction is responsible for the inhibition of the reproduction of a facile crystallization process for a mixture of four isomers which are the products of the Reformatsky reaction. Further, expensive palladium and explosive risk of a high-pressure hydrogen reaction used for the removal of the chlorine substituent restrict industrial application of the production process.

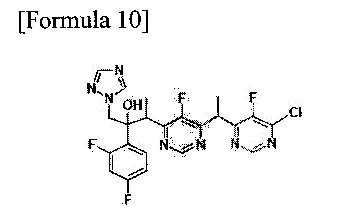

In addition, Korean Patent No. 10-0889937 discloses a process for the preparation of voriconazole using a voriconazole intermediate with substitution of a thio group introduced in place of chloro at the 6-position of 4-(1-bromoethyl)-6-chloro-5-fluoropyrimidine which is a reactant conventionally used in the Reformatsky reaction. However, phenylthio compounds used in the preparation of a phenylthiopyrimidine derivative which is a novel intermediate of this process have a limitation in terms of industrial-scale production due to very irritative and offensive odor inherent thereto.

To this end, in order to search for a thio derivative which does not give off irritative odor and is low in terms of production costs, inventors of the present invention have synthesized and investigated a variety of thio derivatives which do not smell of the odor peculiar to thiols. Unfortunately, with these thio derivative compounds, the Reformatsky reaction using a zinc metal did not proceed and only a debromination reaction took place as a side reaction (Comparative Example 1). *Inter alia,* the present inventors have surprisingly discovered that the compound of formula 2 obtained by the esterification of a thiosalicylic acid derivative is outstandingly high in terms of reactivity of Reformatsky reaction and selectivity of stereoisomers in conjunction with no emission of odor inherent to thiols. The present invention has been completed based on these findings. 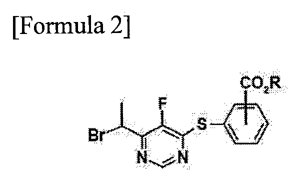 wherein R is C₁-C₆ alkyl or C₃-C₆ cycloalkyl.

### DISCLOSURE OF THE INVENTION

### TECHNICAL PROBLEM

Therefore, the present invention is intended to provide a novel intermediate which is environmentally-friendly and enables the production of voriconazole with high purity and high yield while not causing emission of unique irritative and offensive odor in a production process and a process for the preparation of voriconazole using the same.

### TECHNICAL SOLUTION

The present invention provides a compound represented by formula 2. 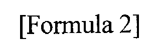 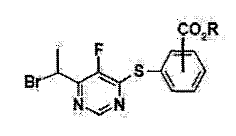 wherein R is C₁-C₆ alkyl or C₃-C₆ cycloalkyl.

A preferred compound of formula 2 is methyl 2-[6-(1-bromo-ethyl)-5-fluoropyrimidin-4-yl-sulfanyl]benzoate.

The compound of formula 2 in accordance with the present invention may be prepared according to the method of Reaction Scheme 3 using 2-mercaptobenzoic acid, 3-mercaptobenzoic acid or 4-mercaptobenzoic acid as a starting material.

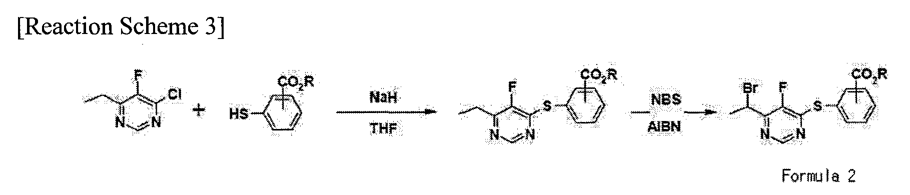

In the above Reaction Scheme, R is C₁-C₆ alkyl or C₃-C₆ cycloalkyl, and preferably methyl.

For example, where the compound of formula 2 is methyl 2-[6-(1-bromo-ethyl)-5-fluoropyrimidin-4-yl-sulfanyl]benzoate, the compound is prepared according to the following method including: a) reacting 2-mercaptobenzoic acid with methanol in the presence of acid to obtain methyl 2-mercaptobenzoate; b) reacting the product of Step a) with 4-chloro-6-ethyl-5-fluoro-pyrimidine in the presence of a base to obtain methyl 2-(6-ethyl-5-fluoropyrimidin-4-yl-sulfanyl)benzoate; and c) reacting the product of Step b) with N-bromosuccinimide and azobisisobutyronitrile to prepare methyl 2-[6-(1-bromo-ethyl)-5-fluoropyrimidin-4-yl-sulfanyl]benzoate.

Further, the present invention provides a process for the preparation of a compound represented by formula 4, including subjecting a compound of formula 2 and 1-(2,4-difluorophenyl)-2-(1H-1,2,4-triazol-1-yl)ethanone to Reformatsky reaction. 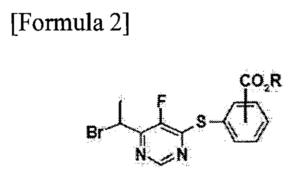 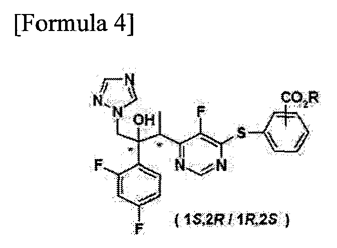 wherein R is C₁-C₆ alkyl or C₃-C₆ cycloalkyl.

In the process for the preparation of the compound of formula 4 in accordance with the present invention, 1-(2,4-difluorophenyl)-2-(1H-1,2,4-triazol-1-yl)ethanone is commercially available or otherwise may be prepared by a known method.

In the process for the preparation of the compound of formula 4 in accordance with the present invention, the Reformatsky reaction is preferably carried out in the presence of activated zinc and iodine or in the presence of activated zinc/lead and iodine. Here, the activated zinc is zinc prepared by treating the commercial zinc powder with an acid aqueous solution, followed by drying. For example, the activated zinc may be in the form of zinc which was washed with dilute hydrochloric acid and then dried.

Lead may be in the form of a powder which is commonly commercially available. The content of the activated zinc is in the range of 3 equivalents to 10 equivalents, and preferably about 8 equivalents, based on 1-(2,4-difluorophenyl)-2-(1H-1,2,4-triazol-1-yl)ethanone. The content of iodine used for initiation of the reaction is in the range of 1 equivalent to 3 equivalents, and preferably about 2 equivalents, based on 1-(2,4-difluorophenyl)-2-(1H-1,2,4-triazol-1-yl)ethanone. Here, the reaction temperature is preferably in the range of 0 to 30□, and the reaction solvent is preferably an ether solvent such as tetrahydrofuran (THF), diethyl ether or 1,4-dioxane, and more preferably THF.

In the process for the preparation of the compound of formula 4 in accordance with the present invention, the enantiomeric pair which is a stereoisomer of the compound of formula 4 is formed in a molar ratio of (1R,2S/1S,2R):(1R,2R/1S,2S) = about 8:2 to about 10:1. At this time, since an undesirable enantiomeric pair (1R,2R/1S,1S) is dissolved to be removed in the solution taking advantage of a difference in physical properties with a preferred enantiomeric pair (1R,2S/1S,2R), the compound of formula 4 which is the preferred enantiomeric pair (1R,2S/1S,2R) can be obtained with a high yield of more than 75%. That is, the difference in physical properties between diastereoisomers means a difference in terms of solubility thereof in a solvent. For example, the compound of formula 4 can be obtained taking advantage of a difference in terms of solubility in a solvent such as ethyl acetate, isopropanol or n-hexane.

Further, the present invention provides a compound of formula 4. 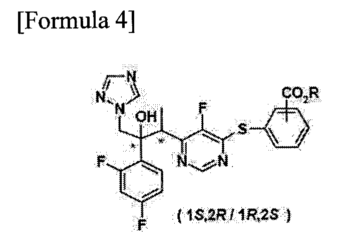 wherein R is C₁-C₆ alkyl or C₃-C₆ cycloalkyl.

The compound of formula 4 in accordance with the present invention is preferably methyl 2-{6-[(1S,2R/1R,2S)-2-(2,4-difluorophenyl)-2-hydroxy-1-methyl-3-[1,2,4]triazo-1-yl-propyl]-5-fluoropyrimidin-4-yl-sulfanyl}benzoate.

Further, the present invention provides a process for the preparation of voriconazole, including a) removing a thiol derivative which is the substituent of the compound of formula 4 in the presence of zinc and organic acid to obtain racemic voriconazole that is (2R,3S/2S,3R)-2-(2,4-difluorophenyl)-3-(5-fluoropyrimidin-4-yl)-1-(1H-1,2,4-triazol-1-yl)butan-2-ol; and b) subjecting the racemic voriconazole which is the product of Step a) to optical resolution with R-(-)-camphorsulfonic acid, thereby obtaining voriconazole which is the compound of formula 1. 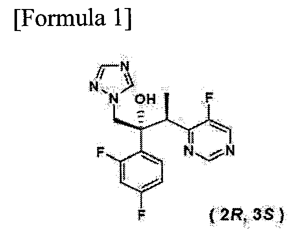 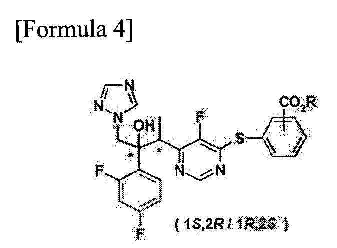 wherein R is C₁-C₆ alkyl or C₃-C₆ cycloalkyl.

In the process for the preparation of voriconazole in accordance with the present invention, the compound of formula 4 is preferably prepared by subjecting a compound of formula 2 and 1-(2,4-difluorophenyl)-2-(1H-1,2,4-triazol-1-yl)ethanone to Reformatsky reaction. 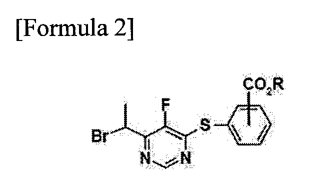 wherein R is C₁-C₆ alkyl or C₃-C₆ cycloalkyl.

In the process for the preparation of voriconazole in accordance with the present invention, the Reformatsky reaction conditions are as mentioned in the process for the preparation of the compound of formula 4 in accordance with the present invention.

In the process for the preparation of voriconazole in accordance with the present invention, the compound of formula 4 is preferably methyl 2-{6-[(1S,2R/1R,2S)-2-(2,4-difluorophenyl)-2-hydroxy-1-methyl-3-[1,2,4]triazo-1-yl-propyl]-5-fluoropyrimidin-4-yl-sulfanyl}benzoate.

In the process for the preparation of voriconazole in accordance with the present invention, the organic acid used in Step a) is preferably selected from ammonium formate, formic acid, ammonium acetate, acetic acid and a mixture thereof and is more preferably ammonium formate.

Here, the content of activated zinc used is preferably 1 equivalents relative to 7 equivalent of the compound of formula 4, and the content of ammonium formate used is preferably 1 equivalents relative to 10 equivalent of the compound of formula 4. The reaction solvent is preferably selected from hydrophilic organic solvents, ethers, ketones and alcohols and is more preferably THF.

In the process for the preparation of voriconazole in accordance with the present invention, optical resolution of the racemic voriconazole of Step a) with R-(-)-camphorsulfonic acid is carried out by a known method described in Korean Patent Application Publication No. 1993-0011039 B1, Korean Patent No. 10-0269048, Korean Patent No. 10-0889937 or the like. For example, racemic voriconazole and R-(-)-10-camphorsulfonic acid are reacted with reflux in an organic solvent to prepare voriconazole R-(-)-10-camphorsulfonic acid, a base such as sodium hydroxide is added to the resulting product, and then voriconazole is separated and obtained.

The process for the preparation of voriconazole in accordance with the present invention is outlined as in Reaction Scheme 4 below.

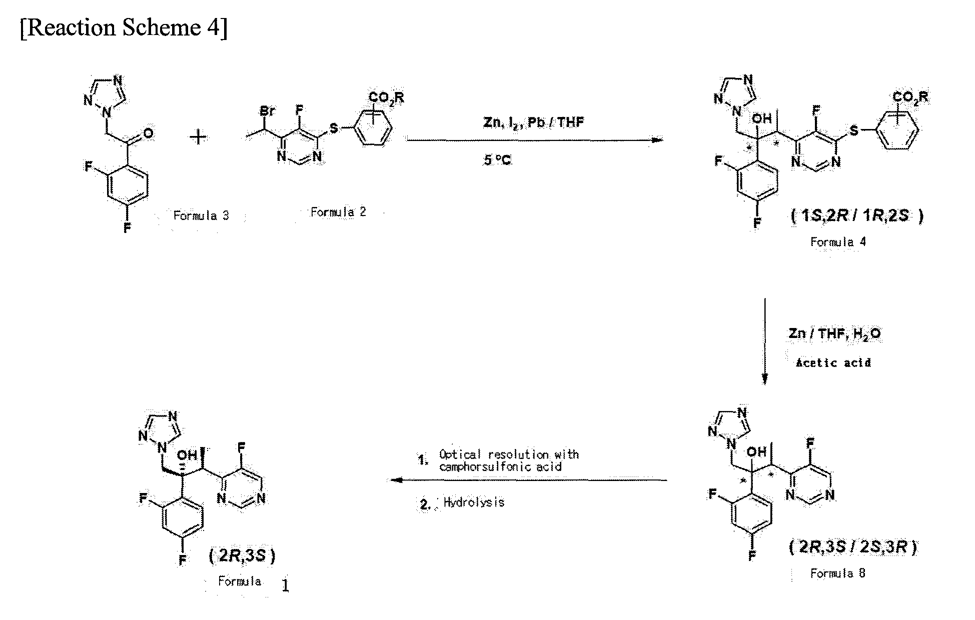

### ADVANTAGEOUS EFFECTS

The process for the preparation of voriconazole in accordance with the present invention using a novel compound of formula 2 and a novel compound of formula 4 provides voriconazole with high purity and high yield while not giving off irritative and offensive odor unique to thiols and is therefore capable of achieving mass production of voriconazole on an industrial scale.

### DESCRIPTION OF THE DRAWINGS

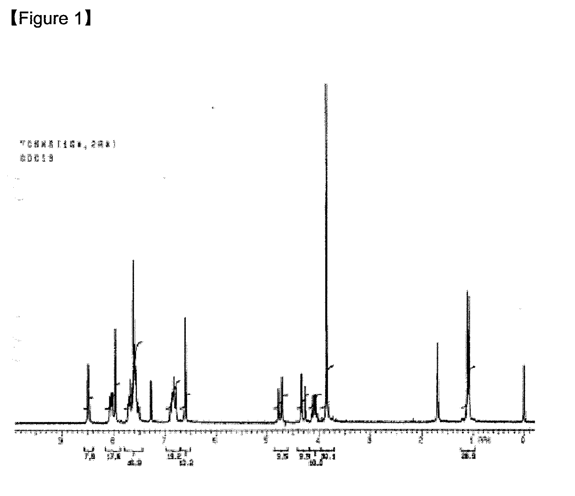

Fig. 1 shows the hydrogen nuclear magnetic resonance spectroscopy (¹H-NMR) spectrum of methyl 2-{6-[(1S,2R/1R,2S)-2-(2,4-difluorophenyl)-2-hydroxy-1-methyl-3-[1,2,4]triazo-1-yl-propyl]-5-fluoropyrimidin-4-yl-sulfanyl}benzoate prepared in Example 2 of the present invention.

### MODE FOR INVENTION

Now, the present invention will be described in more detail with reference to the following Examples, but the present invention is not limited thereto.

Unless otherwise specified, reagents and solvents used in the present invention were purchased from Aldrich, Lancaster, Daejeong Chemical Co., Ltd., Samchun Chemical Co., Ltd., or the like.

In addition, activated zinc referred to hereinafter was a zinc powder (purchased from Daejeong Chemical Co., Ltd.) which was treated according to the method described in the literature [W.L.F. Armarego and D.D., Perrin, "Purification of Laboratory Chemicals", 4th edition, p 452, published by Butterworth Heinemann].

¹H-NMR given hereinafter was measured by using VARIAN GEMINI 200 having a resolution of 200MHz. Chemical purity analysis of products was carried out by HPLC (Model No.: L-2130, manufactured by Hitachi). Ratiometric analysis of an enantiomeric pair was carried out under the following conditions: Column: Kromasil C18 reversed phase column (25cm x 4.6mm, 5µm); Detection wavelength: 254nm; Mobile phase: acetonitrile:water=65:35 (v/v); Flow rate: 1.0mL/min. In addition, ratiometric analysis of stereoisomers was carried out under the following conditions: Chiral column: Daicel OD-H column (25cm x 4.6mm); Detection wavelength: 254nm; Mobile phase: n-hexane:ethanol=7:3(v/v); Flow rate: 1.0mL/min.

### Example 1: Preparation of methyl 2-[6-(1-bromo-ethyl)-5-fluoropyrimidin-4-yl-sulfanyl]benzoate (Preparation of compound of formula 2 with substitution of -CO₂CH₃ at the ortho position)

### Step 1) Preparation of methyl 2-mercaptobenzoate

A mixed solution of 40g of 2-mercaptobenzoic acid and 600mL of methanol was cooled to 5□ and 42mL of sulfuric acid was added dropwise thereto over 10 minutes. After being refluxed for 14 hours, the reaction liquid was concentrated under reduced pressure. The concentrate was diluted with 400mL of methylene chloride and 400mL of purified water and then the organic layer was separated. The organic layer was successively washed with 200mL of a saturated sodium bicarbonate solution and 400mL of brine and then dried over anhydrous sodium sulfate. After the organic layer was concentrated, the resulting residue was subjected to fractional distillation (118□/7mmHg) to afford 42g (yield: 96%) of the title compound as a clear oil.

¹H-NMR (200MHz, CDCl₃) δ (ppm): 8.01 (d,1H), 7.30 (d,2H), 7.14 (m,1H), 4.68 (s,SH), 3.91 (S,3H)

### Step 2) Preparation of methyl 2-(6-ethyl-5-fluoropyrimidin-4-yl-sulfanyl)benzoate

A mixed solution of 31.7g of methyl 2-mercaptobenzoate prepared in Step 1) and 300mL of tetrahydrofuran was cooled to 5□ and 5.0g of sodium hydride was added dropwise thereto over 10 minutes. After being stirred at 5□ for 30 minutes, 30g of 4-chloro-6-ethyl-5-fluoropyrimidine was added dropwise over 20 minutes. The temperature during addition was maintained below 20□. The temperature of the reaction liquid was elevated to 25□, followed by stirring for 30 minutes. The resulting mixture was concentrated under reduced pressure and diluted with 300mL of ethyl acetate. The organic layer was successively washed with 200mL of a saturated ammonium chloride solution and 200mL of purified water. The organic layer was dried over anhydrous sodium sulfate and concentrated under reduced pressure to afford 52.5g (yield: 96%) of the title compound as an oil.

¹H-NMR (200MHz, CDCl₃) δ (ppm): 8.52 (d,1H), 8.01 (m,1H), 7.65-7.50 (m,3H), 3.82 (s,3H), 2.83 (q,2H), 1.32 (t,3H)

### Step 3) Preparation of methyl 2-[6-(1-bromo-ethyl)-5-fluoropyrimidin-4-yl-sulfanyl]benzoate

52.0g of methyl 2-(6-ethyl-5-fluoropyrimidin-4-yl-sulfanyl)benzoate prepared in Step 2), 36.4g of N-bromosuccinimide and 2.9g of azobisisobutyronitrile were dissolved in 500mL of chloroform. The mixture was refluxed for 12 hours. The reaction liquid was cooled to 25□ and successively washed with 500mL of purified water, 500mL of a 5% sodium metabisulfite solution and 300mL of saturated brine. The residue was dried over anhydrous sodium sulfate and concentrated under reduced pressure to afford 60.5g (yield: 91.6%) of the title compound as an oil.

¹H-NMR (200MHz, CDCl₃) δ (ppm): 8.61 (d,1H), 8.03 (m,1H), 7.60-7.53 (m,3H), 5.36 (q,2H), 3.82 (s,3H), 2.05 (d,3H)

### Example 2: Preparation of methyl 2-{6-[(1S,2R/1R,2S)-2-(2,4-difluorophenyl)-2-hydroxy-1-methyl-3-[1,2,4]triazo-1-yl-propyl]-5-fluoropyrimidin-4-yl-sulfanyl}benzoate (Preparation of compound of formula 4 with substitution of -CO₂CH₃ at the ortho position)

34.4g of an activated zinc powder, 2.36g of a lead powder (trade name: LEAD (powder), manufactured by Samchun Chemical Co., Ltd., lead content: 90%) and 240mL of tetrahydrofuran were mixed by stirring and cooled to 5□ in nitrogen gas. To the solution was slowly added a solution of 12.8g of iodine in 100mL of tetrahydrofuran at a temperature of below 45□ over 10 minutes. The resulting solution was cooled to 5□, and a mixed solution of 200mL of tetrahydrofuran, 15g of 1-(2,4-difluorophenyl)-2-(1H-1,2,4-triazol-1-yl)ethanone, 43.4g of methyl 2-[6-(1-bromo-ethyl)-5-fluoropyrimidin-4-yl-sulfanyl]benzoate prepared in Example 1 and 17.6g of iodine was slowly added thereto over 20 minutes. Then, the mixture was warmed to 25□ and stirred for 2 hours, and the solid metal residue was filtered through Celite. Tetrahydrofuran in the solution was removed by distillation under reduced pressure, and the residual solution was diluted with 500mL of ethyl acetate. Thereafter, this solution was washed with 500mL of a saturated ammonium chloride aqueous solution and was successively washed with 500mL of a 5% disodium ethylenediaminetetraacetate dihydrate solution and 500mL of saturated brine. The organic layer was concentrated under reduced pressure, crystallized from 100mL of isopropanol and 100mL of hexane at 25□, filtered and dried to afford 26.9g (yield: 77.6%) of the pale yellow title compound.

¹H-NMR (200MHz, CDCl₃) δ (ppm): 8.49 (s,1H), 8.04 (m,1H), 7.98 (s,1H), 7.70-7.51 (m,4H), 6.79-6.89 (m,2H), 6.60 (s,1H), 4.73 (d,1H), 4.30 (d,1H), 4.08 (q,1H), 3.83 (s,3H), 1.70 (s,OH), 1.08 (d,3H)

In addition, the reaction liquid prior to crystallization was subjected to HPLC analysis using an internal standard material. As a result, the reaction conversion was 95.0% and the ratio of an isomeric pair of the reaction liquid was (1R,2S/1S,2R):(1R,2R/1S,2S)=10:1. The ratio of an enantiomeric pair of the title compound obtained by crystallization was (1R,2S/1S,2R):(1R,2R/1S,2S) =99.6%:0.4%.

### Example 3: Preparation of methyl 2-{6-[(1S,2R/1R,2S)-2-(2,4-difluorophenyl)-2-hydroxy-1-methyl-3-[1,2,4]triazo-1-yl-propyl]-5-fluoropyrimidin-4-yl-sulfanyl}benzoate: no addition of lead powder. (Preparation of compound of formula 4 with substitution of-CO₂CH₃ at the ortho position)

A stirred mixture of 6.9g of an activated zinc powder (trade name: ZINC, manufactured by Daejeong Chemical Co., Ltd., content: 99%) and 48mL of tetrahydrofuran was cooled to 5□ in nitrogen gas. To the solution was slowly added a solution of 2.6g of iodine in 20mL of tetrahydrofuran at a temperature of below 45□ over 10 minutes. This solution was cooled to 5□, and a mixed solution of 40mL of tetrahydrofuran, 3.0g of 1-(2,4-difluorophenyl)-2-(1H-1,2,4-triazol-1-yl)ethanone, 8.7g of methyl 2-[6-(1-bromo-ethyl)-5-fluoropyrimidin-4-yl-sulfanyl]benzoate prepared in Example 1 and 3.5g of iodine was slowly added thereto over 20 minutes. The resulting solution was warmed to 25□ and stirred for 2 hours, and the solid metal residue was filtered through Celite. Tetrahydrofuran in the solution was removed by distillation under reduced pressure, followed by dilution with 100mL of ethyl acetate. This solution was washed with 100mL of a saturated ammonium chloride aqueous solution and was successively washed with 100mL of a 5% disodium ethylenediaminetetraacetate dihydrate solution and 100mL of saturated brine. The organic layer was concentrated under reduced pressure, crystallized from 20mL of isopropanol and 20mL of hexane at 25□, filtered and dried to afford 4.2g (yield: 60.6%) of a pale yellow compound.

The reaction liquid was subjected to HPLC analysis using an internal standard material. As a result, the total conversion was 90.5% and the ratio of an isomeric pair was (1R,2S/1S,2R):(1R,2R/1S,2S)=8:2. The ratio of an enantiomeric pair of the title compound obtained by crystallization was (1R,2S/1S,2R):(1R,2R/1S,2S)= 99.5%:0.5%.

### Example 4: Preparation of (2R,3S/2S,3R)-2-(2,4-difluorophenyl)-3-(5-fluoropyrimidin-4-yl)-1-(1H-1,2,4-triazol-1-yl)butan-2-ol (Preparation of racemic voriconazole)

To 18.7g of an activated zinc powder was added 500mL of tetrahydrofuran, followed by reflux at 64□ for 1 hour. The temperature was lowered and maintained at 50□. To this solution was added 21.0g of methyl 2-{6-[(1S,2R/1R,2S)-2-(2,4-difluorophenyl)-2-hydroxy-1-methyl-3-[1,2,4]triazo-1-yl-propyl]-5-fluoropyrimidin-4-yl-sulfanyl}benzoate prepared in Example 2. 25.7g of ammonium formate in 350mL of water was slowly added thereto over 30 minutes, followed by reflux for 4 hours. After the reaction liquid was cooled to room temperature, the solid metal residue was filtered through Celite and washed with 500mL of ethyl acetate. The filtrate was washed with 300mL of a saturated ammonium chloride aqueous solution and was successively washed with 300mL of sodium bicarbonate, 300mL of a 5% sodium hydroxide aqueous solution and 300mL of brine. The organic layer was dried over anhydrous sodium sulfate, filtered and concentrated. To the concentrate were added 250mL of ethyl acetate and 500mL of n-hexane, followed by stirring for 30 minutes. The resulting solid was removed by filtration. The filtrate was concentrated to afford 15.1g (content: 85%, yield: 90%) of (2R,3S/2S,3R)-2-(2,4-difluorophenyl)-3-(5-fluoropyrimidin-4-yl)-1-(1H-1,2,4-triazol-1-yl)butan-2-ol (hereinafter, referred to as racemic voriconazole) as a crude oil.

### Example 5: Preparation of (2R,3S)-2-(2,4-difluorophenyl)-3-(5-fluoropyrimidin-4-yl)-1-(1H-1,2,4-triazol-1-yl)butan-2-ol R-(-)-10-camphorsulfonate (Preparation of voriconazole R-(-)-10-camphorsulfonate)

15.1g (content: 85%) of racemic voriconazole prepared in Example 4 was dissolved in 288mL of acetone and 8.51g of R-(-)-10-camphorsulfonic acid dissolved in 96mL of methanol was added thereto. This was followed by reflux at 50□ until the reaction liquid became a clear solution, and the reaction liquid was slowly cooled to 20□. The reaction liquid was stirred at 20□ for 18 hours, filtered and dried to afford 7.46g (yield: 35%) of the white title compound as (2R,3S)-2-(2,4-difluorophenyl)-3-(5-fluoropyrimidin-4-yl)-1-(1H-1,2,4-triazol-1-yl)butan-2-ol R-(-)-10-camphorsulfonate (hereinafter, referred to as voriconazole R-(-)-camsylate).

According to the HPLC analysis results, there was no enantiomeric pair of (2R,3R/2S,3S), and an isomeric ratio of the compound was (2R,3S):(2S,3R)=99.9%:0.1%. The optical purity of the product was more than 99.8%.

### Example 6: Preparation of (2R,3S)-2-(2,4-difluorophenyl)-3-(5-fluoropyrimidin-4-yl)-1-(1H-1,2,4-triazol-1-yl)butan-2-ol (Preparation of voriconazole)

7.4g of voriconazole R-(-)-camsylate prepared in Example 5, 50mL of dichloromethane and 50mL of purified water were mixed, and 1.3mL of a 40% sodium hydroxide solution was added thereto. The mixture was adjusted to a pH of 11 to 12. The organic layer was separated, and the aqueous layer was extracted with 15mL of dichloromethane. The combined organic layer was dried over anhydrous sodium sulfate, filtered and concentrated. 13mL of isopropanol was added thereto, followed by stirring at 0□ for 3 hours. The resulting solid was filtered and dried to afford 4.1g (yield: 92%) of the white title compound.

¹H-NMR (200MHz, DMSO-d₆) δ (ppm): 9.04 (s,1H), 8.84 (s,1H), 8.23 (s,1H), 7.62 (s,1H), 7.28 to 7.17 (m,2H), 6.91 (m,1H), 6.02 (bs,1H), 4.80 (d,1H), 4.34 (d,1H), 3.93 (q,1H), 1.1 (d,3H)

According to the HPLC analysis results, the enantiomeric ratio was (2R,3S):(2S,3R)=100%:0%, and the optical purity of the product was more than 99.9%.

### Comparative Example: Reformatsky reaction of 4-(1-bromoethyl)-5-fluoro-6-(1-methyl-1H-tetrazol-5-yl-sulfanyl)pyrimidine

### Step 1: Preparation of 4-ethyl-5-fluoro-6-(1-methyl-1H-tetrazol-5-yl-sulfanyl)pyrimidine

A mixed solution of 14.5g of 5-mercapto-1-methyltetrazole and 200mL of tetrahydrofuran was cooled to 5□ and 3.3g of sodium hydride was added thereto, followed by stirring for 10 minutes. After being stirred at 25□ for 30 minutes, the mixed solution was cooled to 5□. 20g of 4-chloro-6-ethyl-5-fluoropyrimidine was added dropwise thereto at a temperature of below 20□ over 15 minutes, followed by elevation to 25□ and stirring for 30 minutes. The resulting mixture was concentrated under reduced pressure and diluted with 300mL of ethyl acetate. The organic layer was successively washed with 200mL of a saturated ammonium chloride solution and 200mL of purified water. The organic layer was dried over anhydrous sodium sulfate and concentrated under reduced pressure. The concentrate was crystallized from 30mL of isopropanol and 30mL of hexane, filtered and dried to afford 19g (yield: 63.4%) of a pale yellow compound.

¹H-NMR (200MHz, CDCl₃) δ (ppm): 8.52 (s,1H), 4.11 (s,3H), 2.86 (q,2H), 1.32 (t,3H)

### Step 2: Preparation of 4-(1-bromoethyl)-5-fluoro-6-(1-methyl-1H-tetrazol-5-yl-sulfanyl)pyrimidine

10.0g of 4-ethyl-5-fluoro-6-(1-methyl-1H-tetrazol-5-yl-sulfanyl)pyrimidine prepared in Step 1, 11.1g of N-bromosuccinimide and 0.7g of azobisisobutyronitrile were dissolved in 100mL of chloroform, followed by reflux at 62□ for 3 hours. The reaction liquid was cooled to 25□ and successively washed with 100mL of purified water, 100mL of a 5% sodium metabisulfite solution and 100mL of saturated brine. The reaction liquid was dried over anhydrous sodium sulfate and concentrated under reduced pressure. The concentrate was crystallized from 20mL of isopropanol and 10mL of hexane, filtered and dried to afford 11.8g (yield: 89.0%) of a pale yellow compound.

¹H-NMR (200MHz, CDCl₃) δ (ppm): 8.61 (s,1H), 5.34 (q,1H), 4.14 (s,3H), 2.06 (d,3H)

### Step 3: Reformatsky reaction of 4-(1-bromoethyl)-5-fluoro-6-(1-methyl-1H-tetrazol-5-yl-sulfanyl)pyrimidine

A stirred mixture of 1.0g of an activated zinc powder, 0.08g of a lead powder and 10mL of tetrahydrofuran was cooled to 5□ in nitrogen gas. A solution of 0.5g of iodine in 10mL of tetrahydrofuran was slowly added thereto at a temperature of below 45□ over 5 minutes. Then, the reaction mixture was cooled to 5□, and a mixed solution of 10mL of tetrahydrofuran, 0.45g of 1-(2,4-difluorophenyl)-2-(1H-1,2,4-triazol-1-yl)ethanone, 1.27g of 4-(1-bromoethyl)-5-fluoro-6-(1-methyl-1H-tetrazol-5-yl-sulfanyl)pyrimidine prepared in Step 2 and 0.5g of iodine was slowly added thereto over 10 minutes, followed by elevation to 25 □ and stirring for 2 hours. The solid metal residue was filtered through Celite and tetrahydrofuran was removed by distillation, followed by dilution with 40mL of ethyl acetate. The reaction liquid was successively washed with 30mL of a 5% disodium ethylenediaminetetraacetate dihydrate solution and 30mL of saturated brine. The reaction liquid was concentrated under reduced pressure, and the reaction product was separated by column chromatography. The resulting product was 0.9g of 4-ethyl-5-fluoro-6-(1-methyl-1H-tetrazol-5-yl-sulfanyl)pyrimidine and 0.38g of 1-(2,4-difluorophenyl)-2-(1H-1,2,4-triazol-1-yl)ethanone. It was confirmed that Reformatsky reaction using zinc did not proceed and debromination of the starting material 4-(1-bromoethyl)-5-fluoro-6-(1-methyl-1H-tetrazol-5-yl-sulfanyl)pyrimidine took place as a side reaction.

## Claims

1. A compound represented by formula 2. 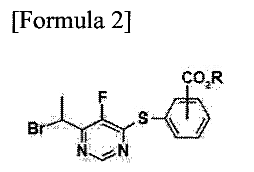 wherein R is C₁-C₆ alkyl or C₃-C₆ cycloalkyl.

2. The compound according to claim 1, wherein the compound of formula 2 is methyl 2-[6-(1-bromo-ethyl)-5-fluoropyrimidin-4-yl-sulfanyl]benzoate.

3. A process for the preparation of a compound represented by formula 4, comprising subjecting a compound of formula 2 and 1-(2,4-difluorophenyl)-2-(1H-1,2,4-triazol-1-yl)ethanone to Reformatsky reaction. 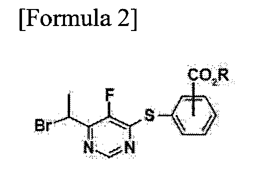 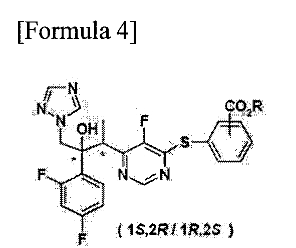 wherein R is C₁-C₆ alkyl or C₃-C₆ cycloalkyl.

4. The process according to claim 3, wherein the Reformatsky reaction is carried out in the presence of activated zinc/lead and iodine.

5. A compound represented by formula 4. 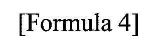 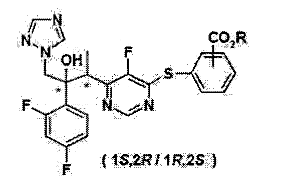 wherein R is C₁-C₆ alkyl or C₃-C₆ cycloalkyl.

6. The compound according to claim 5, wherein the compound of formula 4 is methyl 2-{6-[(1S,2R/1R,2S)-2-(2,4-difluorophenyl)-2-hydroxy-1-methyl-3-[1,2,4]triazo-1-yl-propyl]-5-fluoropyrimidin-4-yl-sulfanyl}benzoate.

7. A process for the preparation of voriconazole, comprising:
a) removing a thiol derivative which is the substituent of the compound of formula 4 in the presence of zinc and organic acid to obtain racemic voriconazole that is (2R,3S/2S,3R)-2-(2,4-difluorophenyl)-3-(5-fluoropyrimidin-4-yl)-1-(1H-1,2,4-triazol-1-yl)butan-2-ol; and
b) subjecting the racemic voriconazole which is the product of Step a) to optical resolution with R-(-)-camphorsulfonic acid, thereby obtaining voriconazole which is the compound of formula 1. 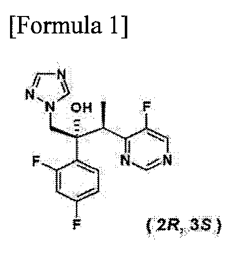 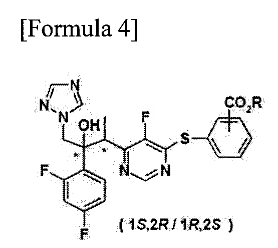 wherein R is C₁-C₆ alkyl or C₃-C₆ cycloalkyl.

8. The process according to claim 7, wherein the organic acid is selected from ammonium formate, formic acid, ammonium acetate, acetic acid and a mixture thereof.

9. The process according to claim 7, wherein the compound of formula 4 is obtained by subjecting a compound of formula 2 and 1-(2,4-difluorophenyl)-2-(1H-1,2,4-triazol-1-yl)ethanone to Reformatsky reaction. 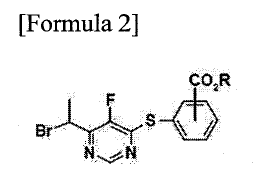 wherein R is C₁-C₆ alkyl or C₃-C₆ cycloalkyl.

10. The process according to claim 9, wherein the Reformatsky reaction is carried out in the presence of activated zinc/lead and iodine.

11. The process according to any one of claims 7 to 10, wherein the compound of formula 4 is methyl 2-{6-[(1S,2R/1R,2S)-2-(2,4-difluorophenyl)-2-hydroxy-1-methyl-3-[1,2,4]triazo-1-yl-propyl]-5-fluoropyrimidin-4-yl-sulfanyl}benzoate.

12. The process according to any one of claims 7 to 10, wherein the removal of a thiol derivative which is the substituent of the compound of formula 4 in Step a) is carried out in the presence of zinc and ammonium formate.
